# EUROPEAN PATENT SPECIFICATION

(11) **EP 0 677 117 B1**
(45) Date of publication and mention of the grant of the patent: **26.05.1999**
(21) Application number: 92915142.1
(22) Date of filing: 08.07.1992
(51) Int. Cl.: C07K 7/00, C07K 17/00, G01N 33/569

(54) **DIFFERENTIATION OF HTLV-I AND HTLV-II USING SYNTHETIC PEPTIDES**
UNTERSCHEIDUNG VON HTLV-I UND HTLV-II UNTER VERWENDUNG VON SYNTHETISCHEN PEPTIDEN
DIFFERENTIATION DE HTLV-I ET HTLV-II A L'AIDE DE PEPTIDES SYNTHETIQUES

(30) Priority: 10.07.1991 US 727765
(43) Date of publication of application: 18.10.1995
(73) Proprietor: ABBOTT LABORATORIES, Abbott Park, IL 60064-2204 (US)
(72) Inventor: SHIH, Jessie, W., Lake Forest, IL 60045 (US); BURCZAK, John, D., Highland Park, IL 60035 (US); LEE, Helen, H., Lake Forest, IL 60045 (US); O'DONNELL, Debra, L., Antioch, IL 60002 (US)
(74) Representative: Modiano, Guido, Dr.-Ing.
(86) International application number: US9205733
(87) International publication number: WO9301316

(56) References cited:
- EP-A- 0 439 077
- EP-A- 0 482 605
- WO-A-89/08664
- WO-A-90/10231
- WO-A-90/15820
- WO-A-92/01713
- WO-A-92/13946
- US-A- 5 003 043
- US-A- 5 283 320
- TRANSFUSION, vol. 31, no. 8suppl., October 1991 page 41S LIPKA ET AL. 'The use of recombinant proteins to differentiate HTLV-I and HTLV-II infections.'
- The Journal of Infectious Diseases, Volume 163, issued January 1990, R.B. LAL et al., "Serologic Discrimination of Human T Cell Lymphotropic Virus Infection by Using a Synthetic Peptide-Based Enzyme Immunoassay", pages 41-46, see the Abstract.
- WO-A-89 08664

## Description

### Background of the Invention.

This invention relates generally to a method useful for the detection of antibodies to Human T-Cell Lymphotropic Virus types I and II (HTLV-I and HTLV-II) in a test sample, and more particularly, relates to synthetic peptides specific for HTLV-I and HTLV-II, respectively, and methods useful for the differential detection of antibodies to HTLV-I and HTLV-II, thereby allowing the differential diagnosis of HTLV-I and HTLV-II infections.

HTLV-I is known to cause disease in humans, whereas HTLV-II is not clearly associated with disease. Epidemiological data indicate that approximately 50% of U.S blood donors confirmed seropositive for HTLV-I are in fact infected with HTLV-II (Lee et al., unpublished observation). Therefore, there is a critical need to be able to distinguish between the two viruses for appropriate donor notification and counseling.

Current screening immunoassays for HTLV-I infection detect antibodies against HTLV-I and to a lesser extent HTLV-II. Serological methods which are more specific than EIA, such as Western blot (WB) and Radioimmunoprecipitation assays (RIPA), cannot distinguish between the two viruses.

To date, differentiation between HTLV-I and HTLV-II is achievable only by use of molecular genetic techniques such as restriction mapping or DNA sequencing of the provirus, and by Polymerase Chain Reaction (PCR) using specific primers for HTLV-I or HTLV-II. These procedures require the use of lymphocytes from patients to be tested which are clearly less convenient to collect and store than serum or plasma test samples. These techniques thus are limited in their usefulness in that they are time consuming, expensive, require specialized facilities and are not easily automated.

United States Patent Nos. 4,525,300 and 4,804,746 to Yoshida teach methods for preparing antibodies to human leukemia virus related peptides. The antibodies disclosed are capable of binding to human leukemia virus.

Palker et al., J. Immunol. 135 (1): 247-254 (1985) report the preparation of monoclonal antibodies reactive with HTLV-I which were raised to synthetic peptides representing sections of the p19 gag internal core protein. Some of the clones generated were reported to bind to HTLV-I virus isolates but not to bind to HTLV-II.

PCT Application No. PCT/US85/01803 to Slamon, published March 27, 1986, teaches a method for the detection of antibodies to HTLV-I and HTLV-II in samples by means of incubating samples with synthetic or cloned polypeptides and proteins derived from the HTLV genome and immobilized on a solid support. The teachings include methods for differentially diagnosing HTLV-I and HTLV-II, which require immunoprecipitation of proteins followed by molecular mass determination by methods such as SDS PAGE electrophoresis. However, SDS PAGE electrophoresis is not easily automated or convertible to a form suitable for routine laboratory use.

U.S. Patent No. 4,689,398 to Wu teaches a further group of synthetic peptides, derived from the HTLV genome sequence, which may be used to detect antibodies specific to HTLV in test samples.

European Patent Application No. 0 267 622 to Masanori, published May 18, 1988, teaches a device comprising a fused HTLV gag and env gene protein immobilized on a solid phase which may be used to detect antibodies to these proteins in a sample. However, this device is unable to distinguish between antibodies to HTLV-I and HTLV-II.

Palker et al. J. Immunol. 142:971-978 (1989) report the mapping of the immunogenic regions of the HTLV-I gp46 and gp21 env proteins and the synthesis of peptides which are useful in the generation of specific monoclonal antibodies. These peptides may be used in immunoassays to detect antibodies to HTLV. Additionally, the report suggests the presence of, but does not identify, a region of the gp46 protein which is not shared by HTLV-I and HTLV-II and may therefore be used to differentially detect antibodies to the two viruses.

PCT Publication No. W089/08664 (PCT/SE89/00126) to Vahine et al., published September 21, 1989, teaches of further synthetic peptides, derived from the env region of the HTLV-I genome, which may be used in the detection of antibodies to the HTLV-I virus. No mention is made of differentiation between antibodies against HTLV-I and HTLV-II.

PCT Publication No. WO90/08162 to United Biomedical Inc., published July 26, 1990, describes synthetic peptides for the detection of HTLV-I reactive antibodies and diagnosis of ATL (adult T cell leukemia/lymphoma). These peptides are from the transmembrane (p21e) and external (gp46) segments of the envelope protein of HTLV-I. Also described are immunoassays using these peptides. The peptide(s) described are used in the SynthEIA® (Olympus Corp., Lake Success, NY) for HTLV-I.

PCT Publication No. WO 90/10231 to Blomberg, published March 5, 1990, teaches a method for differentially detecting antibodies to HTLV-I and HTLV-II by detecting binding of such antibodies to synthetic peptides derived from the gag and env regions of HTLV-I and HTLV-II. The method described requires the performance of at least four immunoassays on each sample and would therefore be inconvenient for the routine screening of a large number of samples.

PCT publication No. W090/15820 to Vahlne et al., published December 27, 1990, describes peptides and antibodies derived from the disclosed peptides which are immunologically reactive with HTLV-I specific antibodies. Several of the peptides are capable of distinguishing between HTLV-I and HTLV-II infection.

Recently, R. B. Lal et al. described the serologic discrimination of HTLV-I from HTLV-II using synthetic peptides which could be used to differentiate between HTLV-I and HTLV-II. R. B. Lal et al., J. Infectious Diseases 163:41-46 (January, 1991). In particular, they reported that HTLV-I "Env-5" (amino acids 242-257) represented an immunodominant domain of HTLV-I, and that ENV-5-based ELISA allowed distinction between HTLV-I and HTLV-II. With the exception of this recent article and the two most recent patent applications which describe differentiation, all of the above disclosed techniques are aimed at the detection of specific antibodies to HTLV-I and HTLV-II. To date, however, no detailed methods have been described which would provide a simple method of effectively detecting, and distinguishing between, antibodies to HTLV-I and HTLV-II. In order to detect and distinguish between antibodies against HTLV-I and HTLV-II, unique antigenic determinants on the two viruses must be identified. Antigenic determinants on proteins have been predicted by the identification of hydrophilic regions using the method of Hopp and Woods, Proc. Natl Acad Sci U.S.A 78:3824 (1981) as well as identification of flexible regions using the method of Karplus and Schultz, Naturwissenschaften 72:212-213 (1985). Antigenic determinants appear to be located at hydrophilic as well as flexible regions of protein sequences. Antigenic determinants have also been empirically identified by immunological examination of peptides produced by protein degradation or in vitro synthesis.

It therefore would be advantageous to provide a method to both detect the presence of antibodies to HTLV-I or HTLV-II, and to effectively distinguish between antibodies to each virus. Such a method would be a useful tool to the physician, allowing a more detailed diagnosis of the disease and therefore more appropriate patient counseling and treatment. The method preferably would be in a simple but reproducible form to facilitate routine laboratory usage and generate sensitive and specific results.

### Summary of the Invention

This invention provides a method for differentiating antibodies against HTLV-I from antibodies against HTLV-II in a test sample. A combination of the above described techniques were used to identify antigenic regions in the gp46 env and p19 gag proteins of HTLV-I and HTLV-II. Since the addition or deletion of amino acids to a peptide significantly influences its ability to bind to antibodies, antigenic regions were systematically examined to identify antigenic sequences which are unique and superior to previously described peptides. The invention provides a method comprising (a) determining the presence of antibody against HTLV-I in a test sample by (i) contacting a test sample with at least one peptide specific for HTLV-I, to form a mixture, (ii) incubating the mixture for a time and under conditions sufficient for antigen/antibody complexes to form, (iii) contacting said complexes with an indicator reagent comprising a signal generating compound attached to an anti-human IgG antibody to form a second mixture, (iv) incubating said second mixture for a time and under conditions sufficient for antigen/antibody/antibody complexes to form, (v) determining the presence of antibody against HTLV-I by detecting the measurable signal; (b) determining the presence of antibodies against HTLV-II in the test sample, by (i) contacting a test sample with at least one peptide specific for HTLV-II, to form a mixture, (ii) incubating the mixture for a time and under conditions sufficient for antigen/antibody complexes to form, (iii) contacting said complexes with an indicator reagent comprising a signal generating compound attached to an anti-human IgG antibody, to form a second mixture, (iv) incubating said second mixture for a time and under conditions sufficient for antigen/antibody/antibody complexes to form, (v) determining the presence of antibodies against HTLV-II by detecting the measurable signal; (c) determining the pattern of reaction of the test sample for HTLV-I and HTLV-II to distinguish between HTLV-I and HTLV-II, wherein the peptides specific for HTLV-I include at least one of the following: HTLV-I env-1 (SEQ. ID. NO. 1), HTLV-I env-2 (SEQ. ID. NO. 2), HTLV-I env-3 (SEQ. ID. NO. 3), HTLV-I env-4 (SEQ. ID. NO. 4), HTLV-I env-5 (SEQ. ID. NO. 5), HTLV-I env-6 (SEQ. ID. NO. 6), HTLV-I gag-1 (SEQ. ID. NO. 7), HTLV-I gag-2 (SEQ. ID. NO. 8), HTLV-I gag-3 (SEQ. ID. NO. 9), HTLV-I gag-4 (SEQ. ID. NO. 10), HTLV-I gag-5 (SEQ. ID. NO. 11), HTLV-I gag-6 (SEQ. ID. NO. 12) and HTLV-I gag-7 (SEQ. ID. NO. 13), or the peptides specific for HTLV-II include at least one of the following: HTLV-II env-1 (SEQ. ID. NO. 14), HTLV-II env-1A (SEQ. ID. NO.15), HTLV-II env-2 (SEQ. ID:NO. 16), HTLV-II env-2A (SEQ. ID. NO. 17), HTLV-II env-3 (SEQ. ID. NO. 18), HTLV-II env-4 (SEQ. ID. NO. 19), HTLV-II env-5 (SEQ. ID. NO. 20), HTLV-II env- 5A (SEQ. ID. NO. 21), HTLV-II env-6 (SEQ. ID. NO. 22), HTLV-II env-7 (SEQ. ID. NO. 23), HTLV-II env-8 (SEQ. ID. NO. 24), HTLV-II gag-1 (SEQ. ID. NO. 25), HTLV-II gag-2 (SEQ. ID. NO. 26), HTLV-II gag-3 (SEQ. ID. NO. 27) and HTLV-II gag-4 (SEQ. ID. NO. 28).

The amino acid sequence for the HTLV-I synthetic peptides were obtained from the predicted amino acid sequence as published by Seiki et al., Proc. Natl. Acad. Sci., USA 80:3618-3622 (1983).

The amino acid sequences for the HTLV-II synthetic peptides were obtained from the predicted amino acid sequences of the two HTLV-II prototypes. The amino acid sequence of the Mo HTLV-II prototype was published by Shimotohno et al., Proc. Natl. Acad. Sci. USA 82:3101-3105 (1985). The sequence of the NRA HTLV-II prototype is unpublished data. Peptides HTLV-II env-1 A, HTLV-II env-2A and HTLV-II env 5A are NRA sequences. All the other remaining HTLV-II sequences are Mo sequences.

The invention also provides kits containing the peptides of the invention for differentiating between anti-HTLV-I antibody and anti-HTLV-II antibody.

### Detailed Desciption of The Invention.

The invention provides a method for the detection of antibodies against either HTLV-I or HTLV-II by means of detecting the binding of the antibodies to synthetic peptides.

With the exception of recently published PCT WO90/15820 (cited supra) , previously described peptides for HTLV-I gp46 are in a common region. We identify unique and superior peptide sequences in this region as well as unique peptide sequences in a second antigenic region of HTLV-I env that are specific for HTLV-I and do not cross-react with HTLV-II. The amino acid sequence for the HTLV-I synthetic peptides were obtained from the predicted amino acid sequence as published by Seiki et al., Proc. Nat'l. Acad Sci., USA 80:3618-3622 (1983). We have identified a unique peptide sequence in a previously non-identified region of HTLV-II gp46 env that is specific for HTLV-II, as well as unique and superior peptide sequences in a previously identified antigenic region. The amino acid sequences for the HTLV-II synthetic peptides were obtained from the predicted amino acid sequences of the two HTLV-II prototypes. The amino acid sequence of the Mo HTLV-II prototype was published by Shimotohno et al., Proc. Nat'l, Acad. Sci. USA 82:3101-3105 (1985). The sequence of the NRA HTLV-II prototype is unpublished data. We also have identified unique peptide sequences in antigenic regions of HTLV-I P19 gag and HTLV-II p19 gag that are specific for HTLV-I and HTLV-II, respectively.

In general, the method of the invention comprises contacting a test sample with a solid phase to which at least one of the HTLV-I or HTLV-II peptides are bound, to form a mixture. The mixture is incubated for a time and under conditions sufficient for antigen / antibody complexes to form. Then the complexes are contacted with an indicator reagent comprising an anti-human antibody attached to a signal generating compound, to form a second mixture. The second mixture is incubated for a time and under conditions sufficient to form antigen / antibody / antibody complexes. The presence of immobilized antibody is determined by detecting the measurable signal generated. The investigation of a test sample separately for antibodies against HTLV-I and antibodies against HTLV-II allows an effective method for distinguishing between infections with these two viruses.

The "solid phase" is not critical and can be selected by one skilled in the art. Thus, latex particles, microparticles, beads, membranes, plastic tubes, walls of microtiter wells and tanned sheep red blood cells are all suitable examples. Suitable methods for immobilizing peptides on solid phases include ionic, hydrophobic, covalent interactions and the like. Those skilled in the art will recognize the scope of methodologies which may be applied relative to the application of useful solid phases.

The "test sample" can be a sample of human or animal biological fluid, such as serum, plasma, ascites, urine, cerebral spinal fluid or any other body constituents, or any tissue culture supernatants which might contain the antibodies of interest.

A suitable "indicator reagent" can be a signal generating compound (label) which is capable of generating a measurable signal detectable by external means conjugated (attached) to a specific binding member for antibodies derived from the test sample. In addition to being an antibody member of a specific binding pair for test sample-derived antibodies, the indicator reagent also can be a member of any specific binding pair, including either hapten-anti-hapten such as biotin or anti-biotin, avidin or biotin, a carbohydrate or a lectin, a complementary nucleotide sequence, an effector or a receptor molecule, an enzyme cofactor and an enzyme, an enzyme inhibitor or an enzyme and the like.

The various "signal generating compounds"(labels) contemplated include chromogens, catalysts such as enzymes, luminescent compounds such as fluoroscein and rhodamine, chemiluminescent compounds, radioactive elements, and direct visual labels. Examples of enzymes include alkaline phosphatase, horseradish peroxidase, beta-galactosidase, and the like. The selection of a particular label is not critical, but it will be capable of producing a signal either by itself or in conjunction with one or more additional substances.

In one embodiment of the present invention, at least one of the synthetic peptides of the invention, specific for antibodies against HTLV-I or HTLV-II is immobilized on polystyrene beads. The beads are then incubated with a diluted test sample of human serum, plasma or other body fluid, and incubated under conditions and for an appropriate period of time during which time antibodies will bind specifically to the immobilized peptides on the bead. The bead then is washed to remove any unbound proteins which may be present . A second incubation then is performed in which the bead is incubated with an indicator reagent comprising anti-human antibodies labelled with an appropriate signal generating compound. By means of a suitable detection system, the amount of labelled anti-human antibody complex immobilized on the bead may be determined by measuring the detectable signal. Hence, the presence of specific antibodies against HTLV-I or HTLV-II in the test sample is determined. A comparison of the pattern of reactivity of the test sample to antibodies against HTLV-I and antibodies against HTLV-II allows distinction between infections with the two viruses.

In a second embodiment of the present invention, at least one of the synthetic peptides of the invention specific for HTLV-I or HTLV-II is immobilized on polystyrene beads. The beads are then incubated with a test sample or a diluted test sample and an appropriate indicator reagent comprising signal generating compound attached to anti-human IgG, under conditions and for an appropriate period of time, during which time antibodies will bind specifically to the immobilized peptides on the bead and simultaneously, thus bind to the indicator reagent. The amount of labelled anti-human antibody complex immobilized on the bead may be determined by detecting the measurable signal generated. Thus, the presence of antibodies against HTLV-I or HTLV-II in the test sample can be determined by a single incubation. A comparison of the pattern of reactivity of the test sample to antibodies against HTLV-I and antibodies against HTLV-II allows distinction between infections with the two viruses.

In a third embodiment of the present invention, at least one of the peptides of the invention specific for antibodies against HTLV-I or antibodies against HTLV-II, are immobilized on a nitrocellulose membrane. The peptide also can be conjugated or crosslinked to itself, other peptides or to various carrier proteins such as BSA, keyhole limpet hemocyanin, ovalbumin, and the like, before immobilization on the nitrocellulose membrane. The test sample is diluted and incubated on the membrane for a time and for conditions sufficient for antigen / antibody complexes to form. The membrane surface is then washed to remove unbound proteins, and in a second incubation, the membrane is incubated with an indicator reagent comprising anti-human antibodies labelled with a signal generating compound. The amount of labelled anti-human antibody immobilized on the membrane, thus the presence of antibodies against either HTLV-I or HTLV-II, is determined by a detecting the measurable signal generated with suitable detection system. Quantification of the level of signal recognized by the detection system allows the quantification of the amount of specific antibody present in a test sample. A comparison of the pattern of reactivity of the test sample to antibodies against HTLV-I and antibodies against HTLV-II allows distinction between infections with the two viruses.

Other embodiments which utililize various other solid phases also are contemplated and are within the scope of this invention. For example, ion capture procedures for immobilizing an immobilizable reaction complex with a negatively charged polymer, described in co-pending U. S. Patent Application Serial No. 150,278 corresponding to EP publication 0326100, and U. S. Patent Application Serial No. 375,029 (EP publication no. 0406473) both of which enjoy common ownership, can be employed according to the present invention to effect a fast solution-phase immunochemical reaction. An immobilizable immune complex is separated from the rest of the reaction mixture by ionic interactions between the negatively charged poly-anion/immune complex and the previously treated, positively charged porous matrix and detected by using various signal generating systems previously described, including those described in chemiluminescent signal measurements as described in co-pending U.S. Patent Application Serial No.921,979 corresponding to EPO Publication No. 0 273,115, which enjoys common ownership.

Also, the methods of the present invention can be adapted for use in systems which utilize microparticle technology including in automated and semi-automated systems wherein the solid phase comprises a microparticle. Such systems include those described in pending U. S. Patent Applications 425,651 and 425,643, which correspond to published EPO applications Nos. EP 0 425 633 and EP 0 424 634, respectively.

The use of scanning tunnelling microscopy for immunoassays also is a technology to which the methods of the present invention are easily adaptable. In scanning probe microscopy, in particular in atomic force microscopy, the capture phase, for example, a selected peptide or peptides of the invention, is adhered to a solid phase and a scanning probe microscope is utilized to detect antigen/antibody complexes which may be present on the surface of the solid phase. The use of scanning tunnelling microscopy eliminates the need for labels which normally must be utilized in many immunoassay systems to detect antigen/antibody complexes. Such a system is described in pending U. S. patent application Serial No. 662,147, which enjoys common ownership.

While the present invention discloses the preference for the use of solid phases, it is contemplated that the peptides of the present invention can be utilized in non-solid phase assay systems. These assay systems are known to those skilled in the art, and are considered to be within the scope of the present invention.

We have been able to design tests which detect specific antibodies against HTLV-I separately from antibodies against HTLV-II by selecting appropnate synthetic peptides of each of these viruses, and coating them onto solid phases, thereby facilitating the differential diagnosis of the two viral infections. Peptides suitable for the specific detection antibodies against HTLV-I are specified herein and comprise the peptides of the HTLV-I env region and the peptides of the HTLV-I gag region which are designated as SEQ. ID. No. 1 through SEQ. ID. NO. 13. Peptides suitable for the specific detection of antibodies against HTLV-II also are specified herein and comprise the peptides of the HTLV-II env region, and the peptides of the HTLV-II gag region, and are designated as SEQ. ID. NO. 14 through SEQ. ID. NO. 28.

It is contemplated that the reagent employed for the assay can be provided in the form of a kit with one or more containers such as vials or bottles, with each container or vial containing a separate reagent such as a diluent, indicator reagent, signal generating compound, assay reagents comprising at least one peptide of the present invention, and the like.

The present invention will now be described by way of examples, which are meant to illustrate, but not to limit, the spirit and scope of the invention.

### EXAMPLES

### Example 1

### Detection of Antibodies against HTLV-I

### Reagents

Peptides were synthesized by stepwise addition of amino acids to a solid phase using procedures known in the art and described in Merrifield J. Am.Chem Soc 85: 2149-2154 (1963) and Barany and Merrifield in E. Gross and J. Meienhofer, eds., The Peptides, Vol. 2:1-284(1979), Academic Press, New York, which are incorporated herein by reference. Briefly, the procedure was as follows. The synthesis was performing starting with the C terminus and progressing to the N terminus. N-protected amino acids were used, with N being t-butyloxycarbonyl (t-Boc). The C terminus t-Boc amino acid was attached to the solid phase. The t-Boc protecting group was removed with trifluoroacetic acid, leaving a free amino group to couple to the next amino acid. Successive t-Boc amino acids were added, coupled using a reagent such as DCC (N-N'-dicyclohexylcarbodiimide) and then deprotected. Once the peptide was completed, the final t-Boc protecting group was removed and the peptide was cleaved from the polymer by using anhydrous hydrogen fluoride.

Peptides prepared as described hereinabove were coated on polystyrene beads as the solid support for capture of antibodies against HTLV-I using procedures known in the art . Briefly, the polystyrene beads were washed with distilled water and incubated at 40°C for two (2) hours with between 0.01µg/ml and 50 µg/ml of peptide(s) in a phosphate buffered saline (PBS) solution. The beads were washed once with PBS containing 0.1% Triton X-100® for one (1) hour, blocked for one (1) hour with 2% bovine serum albumin (BSA) in PBS, overcoated with 5% sucrose in PBS for 15 minutes, and then dried.

Beads used in this example as the solid phase for the detection of antibodies to HTLV-I were co-coated with peptide HTLV-I env-1 (SEQ. ID NO. 1) corresponding to amino acids 174-204 and peptide HTLV-I env-4 (SEQ. ID NO. 5) corresponding to amino acids 237-260 of the HTLV-I gp46 env protein.

Anti-human lgG antiserum was prepared by immunizing goats with purified human IgG, according to known methods. The resulting antiserum was affinity purified and labelled with Horseradish Peroxidase (HRPO).

### Method

Samples were diluted in sample diluent buffer between dilution factors of 1:2 and 1:1000. 200 µl of the diluted sample was incubated with a coated bead in a reaction tray for 60 minutes at 40°C. After thorough washing, the beads were incubated with goat anti-human HRPO diluted in a suitable diluent, for 30 minutes at 40 °C. The beads were again thoroughly washed. The amount of HRPO immobilized on the beads was quantified by incubating with an O-phenylenediamine:2HCl (OPD) reagent for 30 minutes at ambient room temperature. At the end of this incubation 1.0 ml of 1 N sulfuric acid was added to stop the color generating reaction. The degree of color generation was determined by measuring the absorbance of the resulting solution at 492/600nm.

### Results

When the method of the invention was applied to a panel of test samples that previously has been confirmed to be positive for antibodies against HTLV by the method of D.W. Anderson et al., Blood 74:2585-2591 (1989) and confirmed HTLV-I positive by PCR, the method of the invention detected 55 of 57 (96.5%) samples positive for antibodies against HTLV-I.

### Example 2

### Detection of Antibodies against HTLV-II

### Reagents

Peptides prepared as described hereinabove in Example 1 were coated on polystyrene beads as the solid phase for capture of antibodies against HTLV-II, as follows. The polystyrene beads were washed with 15% v/v isopropanol and incubated at 40°C for two (2) hours with between 0.01µg/ml and 50 µg/ml of peptide(s) in a phosphate buffered saline (PBS) solution. The beads were washed once with PBS containing 0.15% Triton X-100® for one hour and blocked for one hour with 2% bovine serum albumin (BSA) in PBS, and then overcoated with 5% sucrose in PBS for 20 minutes at room temperature and then dried.

For the detection of antibodies against HTLV-II in this example, beads were coated with peptide HTLV-II env-2 (SEQ. ID NO. 16) corresponding to amino acids 171-198 of the HTLV-II gp46 env protein.

Anti-human IgG antiserum was prepared by immunizing goats with purified human IgG. The resulting antiserum was affinity purified and labelled with Horseradish Peroxidase (HRPO) according to standard methods known in the art.

### Method

Samples were diluted in sample diluent buffer between dilution factors of 1:2 and 1:1000. 200 µl of the diluted sample was incubated with a coated bead in a reaction tray for 60 minutes at 40°C. After thorough washing, the beads were incubated for 30 minutes at 40°C with goat anti-human HRPO diluted in a suitable diluent. The beads were again thoroughly washed. The amount of HRPO immobilized on the bead was quantified by incubating with an O-phenylenediamine:2HCl (OPD) reagent for 30 minutes at ambient room temperature. At the end of this incubation, 1.0 ml of 1 N sulfuric acid was added to stop the color generating reaction. The degree of color generation was determined by measuring the absorbance of the substrate at 492/600nm.

### Results

When this method was applied to a panel of test samples which had been confirmed to be positive for antibodies against HTLV by the method of Anderson (cited supra) and positive for HTLV-II by PCR, the test was able to detect 72 of 74 samples (97.3%).

### Example 3

### Detection of Antibodies Against HTLV-I or HTLV-II

Reagents were prepared as for example 2 except that peptides HTLV-I gag-1 (SEQ. ID. NO. 7), HTLV-II gag3 (SEQ. ID. NO. 27), HTLV-II env-8 (SEQ, ID. NO. 24) and HTLV-I env-1 (SEQ. ID, NO. 1) were individually coated onto beads. The beads then were used in the assay method of the invention following the procedure of Examples 1 and 2. The data presented below in Table 1 is a compilation of the data generated in examples 1,2 and 3.

It also was shown that by using the peptides of examples 1,2 and 3, with the method of the invention, we were able to detect 57/57 (100%) test samples confirmed to be positive for antibodies against HTLV-I, In addition, the method of the invention was able to detect 73 / 74 (98.6%) of samples confirmed to be positive for antibodies against HTLV-II.

### Example 4

### Comparison of Present Invention With Other Tests.

The methods of examples 1,2 and 3 were used to investigate a panel of test samples which had previously been classified by Polymerase Chain Reaction (PCR). The present invention was compared with three other methods (PCR, SynthEIA^{™}[available from Olympus Corp., Lake Success, NY] and a method by Svennerholm [cited supra as Vahlne, EP Publication No. WO89/08664]) designed to detect, and to discriminate between, antibodies against HTLV-I and HTLV-II. The Svennerholm peptides tested were HTLV-I H, O, T, V, and HTLV-II H, O, and T. The Blomberg peptides tested were the four disclosed preferred peptides (1GB, 2GB, 1EA and 2EA), in approximately the same region as the peptides of this disclosure. Each of the comparative methods were conducted according to the disclosed preferred method, or in the case of the commercially available SynthEIA, according to the manufacturers' recommended protocol. The results of the comparison are shown in Tables 1 and 2.

**TABLE 1**

| Comparison of Disclosed Method With Other Tests. | | | | |
|---|---|---|---|---|
| Confirmation Status | PCR Result | Method | | |
| | | Present Method | SynthEIA | Svennerholm┼ |
| Confirmed | HTLV-I | 57/57 (100%) | 10/14 (71.4%) | 8/11 (72.7%)* |
| Confirmed | HTLV-II | 73/ 74 (98.6%) | 9/31 (29.0%) | 5/12 (41.7%) |
| Indeterminate | HTLV-I | 6/8 (75%) | 1/8 (12.5%) | 1/3 (33.3%) |
| Indeterminate | HTLV-II | 3/9 (33.3%) | 1/9 (11.1%) | 0/2 (0%) |
| Seronegative | | 0/144 (0%) | | 5/ 44 (11.4%) |

| | | | | |
|---|---|---|---|---|
| * One sample discrepent with PCR results; ┼previously cited. | | | | |

**TABLE 2**

| Comparison of Disclosed Method With The Method of Blombergt | | | | | |
|---|---|---|---|---|---|
| Confirmation Status | PCR Result | Method | | | |
| | | Present | Method | Blomberg | |
| | | env | gag | env | gag |
| Confirmed | HTLV-I | 48/50 | 49/ 50 | 43/50 | 36/50 |
| | | (96%) | (98%) | (86%) | (72%) |
| Confirmed | HTLV-II | 47/50 | 35/50* | 34/50 | 40/50* |
| | | (94%) | (68%) | (68%) | (80%) |

| | | | | | |
|---|---|---|---|---|---|
| * In the present method, two HTLV-I samples cross-reacted with this peptide. In the Blomberg method, 24 HTLV-I samples cross-reacted; †previously cited. | | | | | |

Using the method described in this example, we have demonstrated the serological differentiation of antibodies against HTLV-I from antibodies against HTLV-II using a panel of highly pedigreed samples. When compared with existing methods, which use peptides with other sequences, the peptides described herein show superior sensitivity and specificity. In comparison to the Polymerase Chain Reaction (PCR) reference method, the described method of the invention provides high efficacy and much improved ease of use.

### Example 5

### Effect of Peptide Sequence on Assay Performance

Reagents were prepared as for examples 1,2 and 3 except that beads were coated individually with HTLV-I env-6 (SEQ. ID. NO. 6) (190-213), HTLV-I gag-4 (SEQ. ID. NO. 10) (103-116), HTLV-I gag-3 (SEQ. ID. NO. 9) (109-129) and HTLV-II env-7 (SEQ. ID. NO. 23) (186-195). The data from these experiments, performed as the methods of examples 1,2 and 3, was tabulated (Table 3) and compared to the data previously generated for experiments 1,2 and 3.

**TABLE 3**

| Comparison Of Peptide Amino Acid Sequences and Serological Reactivities | | | |
|---|---|---|---|
| Protein | Amino Acids | HTLV-I Reactivity | HTLV-II Reactivity |
| HTLV-I gp46 | 190-213 | 4/13 | |
| HTLV-I gp46 | 174-204 | 13/13 | |
| | | | |
| HTLV-II gp46 | 186-195 | | 0/9 |
| HTLV-II gp46 | 171-198 | | 9/9 |
| | | | |
| HTLV-I p19 | 103-116 | 0/14 | |
| HTLV-I p19 | 100-129 | 14/14 | |
| HTLV-I p19 | 109-129 | 5/14 | |

This data clearly demonstrates that the addition, or removal, of small numbers of amino acids to or from a peptide may significantly influence its ability to bind to an antibody.

### Example 6

### Reactivity of Peptides

Reagents were prepared as for example 2 except that peptides SEQ. ID 1 THROUGH SEQ. ID 28 were individually coated onto beads. The data presented below in Table 4 is a compilation of the data generated . The corresponding designation of the SEQ. ID. No. is indicated in parenthesis next to the SEQ. ID. NO.

It was shown that by using the peptides SEQ. ID 1 through SEQ. ID 13 with the method of the invention, we were able to detect 14/14 (100%) test samples confirmed to be positive for antibodies against HTLV-I, with no cross-reactivity observed for HTLV-II. In addition the method of the invention was able to detect 9/14 (64.3%) of samples confirmed to be positive for antibodies against HTLV-II by using a single peptide selected from SEQ. ID 14 through SEQ. ID 28. Cross-reactivity was observed with only one peptide of the group SEQ. ID 14 to SEQ. ID. 28: SEQ. ID. 25.

**TABLE 4**

| Reactivity of Individual Peptides | | | |
|---|---|---|---|
| Peptide | Amino Acid | HTLV-I Reactivity | HTLV-II Reactivity |
| SEQ. ID. 1 ( HTLV-I env-1) | 174-204 | 11/14 | 0/14 |
| SEQ. ID. 2 (HTLV-I env-2) | 180-213 | 13/14 | 0/14 |
| SEQ. ID. 3 (HTLV-I env-3) | 227-257 | 7/14 | 0/14 |
| SEQ. ID. 4 (HTLV-I env-4) | 230-260 | 12/14 | 0/14 |
| SEQ. ID. 5 (HTLV-I env-5) | 237-260 | 9/14 | 0/14 |
| SEQ. ID. 6 (HTLV-I env-6) | 190-213 | 4/14 | 0/14 |
| SEQ. ID. 7 (HTLV-I gag-1) | 100-129 | 14/14 | 0/14 |
| SEQ. ID. 8 (HTLV-I gag-2) | 104-129 | 14/14 | 0/14 |
| SEQ. ID. 9 (HTLV-I gag-3) | 109-129 | 5/14 | 0/14 |
| SEQ. ID. 10 (HTLV-I gag-4) | 103-116 | 0/14 | 0/14 |
| SEQ. ID 11 (HTLV-I gag-5) | 100-119 | 10/14 | 0/14 |
| SEQ. ID 12 (HTLV-I gag-6) | 100-127 | 13/14 | 0/ 14 |
| SEQ. ID 13 (HTLV-I gag-7) | 100-126 | 13/14 | 0/14 |
| | | | |
| SEQ. ID. 14 (HTLV-II env-1) | 167-198 | 0/14 | 8/14 |
| SEQ. ID. 15 (HTLV-II env-1A)* | 167-198 | 0/14 | 9/14 |
| SEQ. ID. 16 (HTLV-II env-2) | 171-198 | 0/14 | 6/14 |
| SEQ. ID. 17 (HTLV-II env-2A)* | 171-198 | 0/14 | 9/14 |
| SEQ. ID. 18 (HTLV-II env-3) | 173-200 | 0/14 | 5/14 |
| SEQ. ID. 19 (HTLV-II env-4) | 173-204 | 0/14 | 8/14 |
| SEQ. ID. 20 (HTLV-II env-5) | 176-209 | 0/14 | 4/14 |
| SEQ. ID. 21 (HTLV-II env-5A)* | 176-209 | 0/14 | 9/14 |
| SEQ. ID. 22 (HTLV-II env-6) | 228-257 | 0/14 | 3/14 |
| SEQ. ID. 23 (HTLV-II env-7) | 186-195 | 0/14 | 0/14 |
| SEQ. ID. 24 (HTLV-II env-8) | 83-108 | 0/14 | 5/14 |
| SEQ. ID. 25 (HTLV-II gag-1) | 115-135 | 6/14 | 3/14 |
| SEQ. ID. 26 (HTLV-II gag-2) | 111-129 | 0/14 | 4/14 |
| SEQ. ID. 27 (HTLV-II gag-3) | 109-127 | 0/14 | 5/14 |
| SEQ. ID. 28 (HTLV-II gag-4) | 107-125 | 0/14 | 2/ 14 |

| | | | |
|---|---|---|---|
| * These three sequences were derived from the NRA HTLV-II prototype. | | | |

Other variations and modifications of the specific embodiments of the invention as set forth herein will be apparent to those skilled in the art. Accordingly, the invention is intended to be limited only in accordance with the appended claims.

## Claims

1. A method for differentiating antibodies against HTLV-I from antibodies against HTLV-II in a test sample, comprising:
a. determining the presence of antibodies against HTLV-I in a test sample, said determination comprising:
i. contacting a test sample with at least one peptide specific for HTLV-I, to form a mixture;
ii. incubating the mixture for a time and under conditions sufficient for antigen/antibody complexes to form;
iii. contacting said complexes with an indicator reagent comprising a signal generating compound attached to an anti-human IgG antibody, to form a second mixture;
iv. incubating said second mixture for a time and under conditions sufficient for antigen/antibody/antibody complexes to form;
v. determining the presence of antibodies against HTLV-I by detecting the measurable signal;
b. determining the presence of antibodies against HTLV-II in a test sample, said determination comprising:
i. contacting a test sample with at least one peptide specific for HTLV-II, to form a mixture;
ii. incubating the mixture for a time and under conditions sufficient for antigen/antibody complexes to form;
iii. contacting said complexes with an indicator reagent comprising a signal generating compound attached to an anti-human IgG antibody, to form a second mixture;
iv. incubating said second mixture for a time and under conditions sufficient for antigen/antibody/antibody complexes to form;
v. determining the presence of antibodies against HTLV-II by detecting the measurable signal;
c. determining the pattern of reaction of the test sample for antibodies against HTLV-I and HTLV-II to distinguish between HTLV-I and HTLV-II infections, wherein said peptide specific for HTLV-I comprises at least one peptide selected from the group consisting of SEQ. ID. NO. 1, SEQ. ID. NO. 2, SEQ. ID. NO. 3, SEQ. ID. NO. 4, SEQ. ID. NO. 5, SEQ. ID. NO. 6, SEQ. ID. NO. 7, SEQ. ID. NO. 8, SEQ. ID. NO. 9, SEQ. ID. NO. 10, SEQ. ID. NO. 11, SEQ. ID. NO. 12 and SEQ. ID. NO. 13.

2. A method for differentiating antibodies against HTLV-I from antibodies against HTLV-II in a test sample, comprising:
a. determining the presence of antibodies against HTLV-I in a test sample, said determination comprising:
i. contacting a test sample with at least one peptide specific for HTLV-I, to form a mixture;
ii. incubating the mixture for a time and under conditions sufficient for antigen/antibody complexes to form;
iii. contacting said complexes with an indicator reagent comprising a signal generating compound attached to an anti-human IgG antibody, to form a second mixture;
iv. incubating said second mixture for a time and under conditions sufficient for antigen/antibody/antibody complexes to form;
v. determining the presence of antibodies against HTLV-I by detecting the measurable signal;
b. determining the presence of antibodies against HTLV-II in a test sample, said determination comprising:
i. contacting a test sample with at least one peptide specific for HTLV-II, to form a mixture;
ii. incubating the mixture for a time and under conditions sufficient for antigen/antibody complexes to form;
iii. contacting said complexes with an indicator reagent comprising a signal generating compound attached to an anti-human IgG antibody, to form a second mixture;
iv. incubating said second mixture for a time and under conditions sufficient for antigen/antibody/antibody complexes to form;
v. determining the presence of antibodies against HTLV-II by detecting the measurable signal;
c. determining the pattern of reaction of the test sample for antibodies against HTLV-I and HTLV-II to distinguish between HTLV-I and HTLV-II infections, wherein said peptide specific for HTLV-II comprises at least one peptide selected from the group consisting of SEQ. ID. NO. 14, SEQ. ID. NO. 15, SEQ. ID. NO. 16, SEQ. ID. NO. 17, SEQ. ID. NO. 18, SEQ. ID. NO. 19, SEQ. ID. NO. 20, SEQ. ID. NO. 21, SEQ. ID. NO. 22, SEQ. ID. NO. 23, SEQ. ID. NO. 24, SEQ. ID. NO. 25, SEQ. ID. NO. 26, SEQ. ID. NO. 27 and SEQ. ID. NO. 28.

3. A method for differentiating antibodies against HTLV-I from antibodies against HTLV-II in a test sample, comprising:
a. determining the presence of antibodies against HTLV-I in a test sample, said determination comprising:
i. contacting a test sample with at least one peptide specific for HTLV-I, to form a mixture;
ii. incubating the mixture for a time and under conditions sufficient for antigen/antibody complexes to form;
iii. contacting said complexes with an indicator reagent comprising a signal generating compound attached to an anti-human IgG antibody, to form a second mixture;
iv. incubating said second mixture for a time and under conditions sufficient for antigen/antibody/antibody complexes to form;
v. determining the presence of antibodies against HTLV-I by detecting the measurable signal;
b. determining the presence of antibodies against HTLV-II in a test sample, said determination comprising:
i. contacting a test sample with at least one peptide specific for HTLV-II, to form a mixture;
ii. incubating the mixture for a time and under conditions sufficient for antigen/antibody complexes to form;
iii. contacting said complexes with an indicator reagent comprising a signal generating compound attached to an anti-human IgG antibody, to form a second mixture;
iv. incubating said second mixture for a time and under conditions sufficient for antigen/antibody/antibody complexes to form;
v. determining the presence of antibodies against HTLV-II by detecting the measurable signal;
c. determining the pattern of reaction of the test sample for antibodies against HTLV-I and HTLV-II to distinguish between HTLV-I and HTLV-II infections, wherein said peptide specific for HTLV-I comprises at least one peptide selected from the group consisting of SEQ. ID. NO. 1, SEQ. ID. NO. 5 and SEQ. ID. NO. 7, and wherein said peptide specific for HTLV-II comprises at least one peptide selected from the group consisting of SEQ. ID. NO. 16, SEQ. ID. NO. 17, SEQ. ID. NO. 24 and SEQ. ID. NO. 27.

4. A peptide specific for HTLV-I having SEQ. ID. NO. 1, SEQ. ID. NO. 5 or SEQ. ID. NO. 7.

5. A peptide specific for HTLV-II having SEQ. ID. NO. 16, SEQ. ID. NO. 17, SEQ. ID. NO. 24 or SEQ. ID. NO. 27.

6. An assay kit for differentiating HTLV-I from HTLV-II, comprising:
a container containing at least one peptide specific for HTLV-I attached to a solid phase; and
a container containing at least one peptide specific for HTLV-II attached to a solid phase,
wherein said peptide specific for HTLV-I comprises at least one peptide selected from the group consisting of SEQ. ID. NO. 1, SEQ. ID. NO. 2, SEQ. ID. NO. 3, SEQ. ID. NO. 4, SEQ. ID. NO. 5, SEQ. ID. NO. 6, SEQ. ID. NO. 7, SEQ. ID. NO. 8, SEQ. ID. NO. 9, SEQ. ID. NO. 10, SEQ. ID. NO. 11, SEQ. ID. NO. 12 and SEQ. ID. NO. 13.

7. An assay kit for differentiating HTLV-I from HTLV-II, comprising:
a container containing at least one peptide specific for HTLV-I attached to a solid phase; and
a container containing at least one peptide specific for HTLV-II attached to a solid phase,
wherein said peptide specific for HTLV-II comprises at least one peptide selected from the group consisting of SEQ. ID. NO. 14, SEQ. ID. NO. 15, SEQ. ID. NO. 16, SEQ. ID. NO. 17, SEQ. ID. NO. 18, SEQ. ID. NO. 19, SEQ. ID. NO. 20, SEQ. ID. NO. 21, SEQ. ID. NO. 22, SEQ. ID. NO. 23, SEQ. ID. NO. 24, SEQ. ID. NO. 25, SEQ. ID. NO. 26, SEQ. ID. NO. 27 and SEQ. ID. NO. 28.

8. An assay kit for differentiating HTLV-I from HTLV-II, comprising:
a container containing at least one peptide specific for HTLV-I attached to a solid phase; and
a container containing at least one peptide specific for HTLV-II attached to a solid phase,
wherein said peptide specific for HTLV-I comprises at least one peptide selected from the group consisting of SEQ. ID. NO. 1, SEQ. ID. NO. 5 and SEQ. ID. NO. 7, and wherein said peptide specific for HTLV-II comprises at least one peptide selected from the group consisting of SEQ. ID. NO. 16, SEQ. ID. NO. 17, SEQ. ID. NO. 24 and SEQ. ID. NO. 27.

## Patentansprüche

1. Verfahren zur Differenzierung von Antikörpern gegen HTLV-I gegenüber Antikörpern gegen HTLV-II in einer Testprobe, das folgendes umfaßt:
a. Bestimmen der Anwesenheit von Antikörpern gegen HTLV-I in einer Testprobe, wobei die Bestimmung folgendes umfaßt:
i. In-Kontakt-Bringen einer Testprobe mit wenigstens einem Peptid, das für HTLV-I spezifisch ist, unter Ausbildung einer Mischung;
ii. Inkubieren der Mischung eine Zeit lang und unter Bedingungen, die zur Ausbildung von Antigen/Antikörper-Komplexen ausreichend sind;
iii. In-Kontakt-Bringen der Komplexe mit einem Indikatorreagenz, das eine signalerzeugende Verbindung umfaßt, die an einen anti-human IgG Antikörper gebunden ist, unter Ausbildung einer zweiten Mischung;
iv. Inkubieren der zweiten Mischung eine Zeit lang und unter Bedingungen, die zur Ausbildung von Antigen/Antikörper/Antikörper-Komplexen ausreichend sind;
v. Bestimmen der Anwesenheit von Antikörpern gegen HTLV-I durch Nachweis des meßbaren Signals;
b. Bestimmen der Anwesenheit von Antikörpern gegen HTLV-II in einer Testprobe, wobei die Bestimmung folgendes umfaßt:
i. In-Kontakt-Bringen einer Testprobe mit wenigstens einem Peptid, das für HTLV-II spezifisch ist, unter Ausbildung einer Mischung;
ii. Inkubation der Mischung eine Zeit lang und unter Bedingungen, die für die Ausbildung von Antigen/Antikörper-Komplexen ausreichend sind;
iii. In-Kontakt-Bringen der Komplexe mit einem Indikatorreagenz, das eine signalerezeugende Verbindung umfaßt, die an einen anti-human IgG Antikörper gebunden ist, unter Ausbildung einer zweiten Mischung;
iv. Inkubation der zweiten Mischung eine Zeit lang und unter Bedingungen, die zur Bildung von Antigen/Antikörper/Antikörper-Komplexen ausreichend sind;
v. Bestimmung der Anwesenheit von Antikörpern gegen HTLV-II durch Nachweis des meßbaren Signals;
c. Bestimmung des Reaktionsmusters der Testprobe gegenüber Antikörpern gegen HTLV-I und HTLV-II, um zwischen HTLV-I und HTLV-II Infektionen zu unterscheiden, worin das für HTLV-I spezifische Peptid wenigstens ein Peptid umfaßt, das aus der Gruppe gewählt ist, die aus folgendem besteht: SEQ.ID.NR. 1, SEQ.ID.NR. 2, SEQ.ID.NR. 3, SEQ.ID.NR. 4, SEQ.ID.NR. 5, SEQ.ID.NR. 6, SEQ.ID.NR. 7, SEQ.ID.NR. 8, SEQ.ID.NR. 9, SEQ.ID.NR. 10, SEQ.ID.NR. 11, SEQ.ID.NR. 12 und SEQ.ID.NR. 13.

2. Verfahren zur Differenzierung von Antikörpern gegen HTLV-I gegenüber Antikörpern gegen HTLV-II in einer Testprobe, das folgendes umfaßt:
a. Bestimmung der Anwesenheit von Antikörpern gegen HTLV-I in einer Testprobe, wobei die Bestimmung folgendes umfaßt:
i. In-Kontakt-Bringen einer Testprobe mit wenigstens einem Peptid, das für HTLV-I spezifisch ist, unter Ausbildung einer Mischung;
ii. Inkubation der Mischung eine Zeit lang und unter Bedingungen, die für die Ausbildung von Antigen/Antikörper-Komplexen ausreichend sind;
iii. In-Kontakt-Bringen der Komplexe mit einem Indikatorreagenz, das eine signalerzeugende Verbindung umfaßt, die an einen anti-human IgG Antikörper angebunden ist, unter Ausbildung einer zweiten Mischung;
iv. Inkubation der zweiten Mischung eine Zeit lang und unter Bedingungen, die für die Ausbildung von Antigen/Antikörper/Antikörper-Komplexen ausreichend sind;
v. Bestimmen der Anwesenheit von Antikörpern gegen HTLV-I durch Nachweis des meßbaren Signals;
b. Bestimmen der Anwesenheit von Antikörpern gegen HTLV-II in einer Testprobe, wobei die Bestimmung folgendes umfaßt:
i. In-Kontakt-Bringen einer Testprobe mit wenigstens einem Peptid, das für HTLV-II spezifisch ist, unter Ausbildung einer Mischung;
ii. Inkubieren der Mischung eine Zeit lang und unter Bedingungen, die für die Ausbildung von Antigen/Antikörper-Komplexen ausreichend sind;
iii. In-Kontakt-Bringen der Komplexe mit einem Indikatorreagenz, das eine signalerzeugende Verbindung umfaßt, die an einen anti-Human IgG Antikörper gebunden ist, unter Ausbildung einer zweiten Mischung;
iv. Inkubation der zweiten Mischung eine Zeit lang und unter Bedingungen, die für die Ausbildung von Antigen/Antikörper/Antikörper-Komplexen ausreichend sind;
v. Bestimmung der Anwesenheit von Antikörpern gegen HTLV-II durch Nachweis des meßbaren Signals;
c. Bestimmung des Reaktionsmusters der Testprobe gegenüber Antikörpern gegen HTLV-I und HTLV-II, um zwischen HTLV-I und HTLV-II Infektionen zu unterscheiden, wobei das für HTLV-II spezifische Peptid wenigstens ein Peptid umfaßt, das aus der Gruppe gewählt ist, die aus folgendem besteht: SEQ.ID.NR. 14, SEQ.ID.NR. 15, SEQ.ID.NR. 16, SEQ.ID.NR. 17, SEQ.ID.NR. 18, SEQ.ID.NR. 19, SEQ.ID.NR. 20, SEQ.ID.NR.21, SEQ.ID.NR. 22, SEQ.ID.NR. 23, SEQ.ID.NR. 24, SEQ.ID.NR. 25, SEQ.ID.NR. 26, SEQ.ID.NR. 27 und SEQ.ID.NR. 28.

3. Verfahren zur Differenzierung von Antikörpern gegen HTLV-I gegenüber Antikörpern gegen HTLV-II in einer Testprobe, das folgendes umfaßt:
a. Bestimmung der Anwesenheit von Antikörpern gegen HTLV-I in einer Testprobe, wobei die Bestimmung folgendes umfaßt:
i. In-Kontakt-Bringen einer Testprobe mit wenigstens einem Peptid, das für HTLV-I spezifisch ist, unter Ausbildung einer Mischung;
ii. Inkubation der Mischung eine Zeit lang und unter Bedingungen, die für die Ausbildung von Antigen/Antikörper-Komplexen ausreichend sind;
iii. In-Kontakt-Bringen der Komplexe mit einem Indikatorreagenz, das eine signalerzeugende Verbindung umfaßt, die an einen anti-human IgG Antikörper angebunden ist, unter Ausbildung einer zweiten Mischung;
iv. Inkubation der zweiten Mischung eine Zeit lang und unter Bedingungen, die für die Ausbildung von Antigen/Antikörper/Antikörper-Komplexen ausreichend sind;
v. Bestimmen der Anwesenheit von Antikörpern gegen HTLV-I durch Nachweis des meßbaren Signals;
b. Bestimmen der Anwesenheit von Antikörpern gegen HTLV-II in einer Testprobe, wobei die Bestimmung folgendes umfaßt:
i. In-Kontakt-Bringen einer Testprobe mit wenigstens einem Peptid, das für HTLV-II spezifisch ist, unter Ausbildung einer Mischung;
ii. Inkubieren der Mischung eine Zeit lang und unter Bedingungen, die für die Ausbildung von Antigen/Antikörper-Komplexen ausreichend sind;
iii. In-Kontakt-Bringen der Komplexe mit einem Indikatorreagenz, das eine signalerzeugende Verbindung umfaßt, die an einen anti-Human IgG Antikörper gebunden ist, unter Ausbildung einer zweiten Mischung;
iv. Inkubation der zweiten Mischung eine Zeit lang und unter Bedingungen, die für die Ausbildung von Antigen/Antikörper/Antikörper-Komplexen ausreichend sind;
v. Bestimmung der Anwesenheit von Antikörpern gegen HTLV-II durch Nachweis des meßbaren Signals;
c. Bestimmung des Reaktionsmusters der Testprobe gegenüber Antikörpern gegen HTLV-I und HTLV-II, um zwischen HTLV-I und HTLV-II Infektionen zu unterscheiden, wobei das für HTLV-I spezifische Peptid wenigstens ein Peptid umfaßt, das aus der Gruppe gewählt ist, die aus folgendem besteht: SEQ.ID.NR. 1, SEQ.ID.NR. 5 und SEQ.ID.NR. 7, und worin das für HTLV-II spezifische Peptid wenigstens ein Peptid umfaßt, das aus der Gruppe gewählt ist, die aus folgendem besteht: SEQ.ID.NR. 16, SEQ.ID.NR. 17, SEQ.ID.NR. 24 und SEQ.ID.NR. 27.

4. Ein Peptid, das für HTLV-I spezifisch ist und die SEQ. ID. NR. 1, SEQ.ID.NR. 5 oder SEQ.ID.NR.7 aufweist.

5. Ein Peptid, das für HTLV-II spezifisch ist und die SEQ.ID.NR. 16, SEQ.ID.NR. 17, SEQ.ID.NR.24 oder SEQ.ID.NR.27 aufweist.

6. Ein Assaykit zur Differenzierung von HTLV-I gegenüber HTLV-II, das folgendes umfaßt:
einen Behälter, der wenigstens ein für HTLV-I spezifisches Peptid enthält, das an eine feste Phase gebunden ist; und
einen Behälter, der wenigstens ein für HTLV-II spezifisches Peptid enthält, das an eine feste Phase gebunden ist,
worin das für HTLV-I spezifische Peptid wenigstens ein Peptid umfaßt, das aus der Gruppe gewählt ist, die aus folgendem besteht: SEQ.ID.NR. 1, SEQ.ID.NR. 2, SEQ.ID.NR. 3, SEQ.ID.NR. 4, SEQ.ID.NR. 5, SEQ.ID.NR. 6, SEQ.ID.NR. 7, SEQ.ID.NR. 8, SEQ.ID.NR. 9, SEQ.ID.NR. 10, SEQ.ID.NR. 11, SEQ.ID.NR. 12 und SEQ.ID.NR. 13.

7. Ein Assaykit zur Differenzierung von HTLV-I gegenüber HTLV-II, das folgendes umfaßt:
einen Behälter, der wenigstens ein für HTLV-I spezifisches Peptid enthält, das an eine feste Phase gebunden ist; und
einen Behälter, der wenigstens ein für HTLV-II spezifisches Peptid enthält, das an eine feste Phase gebunden ist,
worin das für HTLV-II spezifische Peptid wenigstens ein Peptid umfaßt, das aus der Gruppe gewählt ist, die aus folgendem besteht: SEQ.ID.NR. 14, SEQ.ID.NR. 15, SEQ.ID.NR. 16, SEQ.ID.NR. 17, SEQ.ID.NR. 18, SEQ.ID.NR. 19, SEQ.ID.NR. 20, SEQ.ID.NR.21, SEQ.ID.NR. 22, SEQ.ID.NR. 23, SEQ.ID.NR. 24, SEQ.ID.NR. 25, SEQ.ID.NR. 26, SEQ.ID.NR. 27 und SEQ.ID.NR. 28.

8. Ein Assaykit zur Differenzierung von HTLV-I gegenüber HTLV-II, das folgendes umfaßt:
einen Behälter, der wenigstens ein für HTLV-I spezifisches Peptid enthält, das an eine feste Phase gebunden ist; und
einen Behälter, der wenigstens ein für HTLV-II spezifisches Peptid enthält, das an eine feste Phase gebunden ist,
worin das für HTLV-I spezifische Peptid wenigstens ein Peptid umfaßt, das aus der Gruppe gewählt ist, die aus folgendem besteht: SEQ.ID.NR.1, SEQ.ID.NR.5, und SEQ.ID.NR.7, und worin das für HTLV-II spezifische Peptid wenigstens ein Peptid umfaßt, das aus der Gruppe gewählt ist, die aus folgendem besteht: SEQ.ID.NR.16, SEQ.ID.NR.17, SEQ.ID.NR.24, und SEQ.ID.NR.27.

## Revendications

1. Méthode pour différencier les anticorps dirigés contre HTLV-I des anticorps dirigés contre HTLV-II dans un échantillon à tester, comprenant:
a. la détermination de la présence d'anticorps dirigés contre HTLV-I dans l'échantillon à tester, ladite détermination comprenant :
i. la mise en contact de l'échantillon à tester avec au moins un peptide spécifique de HTLV-I, pour former un mélange ;
ii. l'incubation de ce mélange pendant une durée et dans des conditions suffisantes pour que se forment des complexes antigène/anticorps;
iii. la mise en contact desdits complexes avec un réactif indicateur comprenant un composé générant un signal fixé à un anticorps IgG anti-humain, pour former un second mélange ;
iv. l'incubation dudit second mélange pendant une durée et dans des conditions suffisantes pour que se forment des complexes antigène/anticorps/anticorps;
v. la détermination de la présence d'anticorps dirigés contre HTLV-I par la détection du signal mesurable ;
b. la détermination de la présence d'anticorps dirigés contre HTLV-II dans l'échantillon à tester, ladite détermination comprenant :
i. la mise en contact de l'échantillon à tester avec au moins un peptide spécifique de HTLV-II, pour former un mélange ;
ii. l'incubation de ce mélange pendant une durée et dans des conditions suffisantes pour que se forment des complexes antigène/anticorps;
iii. la mise en contact desdits complexes avec un réactif indicateur comprenant un composé générant un signal fixé à un anticorps IgG anti-humain, pour former un second mélange ;
iv. l'incubation dudit second mélange pendant une durée et dans des conditions suffisantes pour que se forment des complexes antigène/anticorps/anticorps;
v. la détermination de la présence d'anticorps dirigés contre HTLV-II par la détection du signal mesurable ;
c. la détermination du schéma réactionnel de l'échantillon à tester pour les anticorps dirigés contre HTLV-I et contre HTLV-II pour faire la distinction entre les infections à HTLV-I et à HTLV-II,
dans laquelle ledit peptide spécifique de HTLV-I comprend au moins un peptide choisi dans le groupe constitué par le n° ID SEQ 1, le n° ID SEQ 2, le n° ID SEQ 3, le n° ID SEQ 4, le n° ID SEQ 5, le n° ID SEQ 6, le n° ID SEQ 7, le n° ID SEQ 8, le n° ID SEQ 9, le n° ID SEQ 10, le n° ID SEQ 11, le n° ID SEQ 12 et le n° ID SEQ 13.

2. Méthode pour différencier les anticorps dirigés contre HTLV-I des anticorps dirigés contre HTLV-II dans un échantillon à tester, comprenant:
a. la détermination de la présence d'anticorps dirigés contre HTLV-I dans l'échantillon à tester, ladite détermination comprenant :
i. la mise en contact de l'échantillon à tester avec au moins un peptide spécifique de HTLV-I, pour former un mélange ;
ii. l'incubation de ce mélange pendant une durée et dans des conditions suffisantes pour que se forment des complexes antigène/anticorps;
iii. la mise en contact desdits complexes avec un réactif indicateur comprenant un composé générant un signal fixé à un anticorps IgG anti-humain, pour former un second mélange ;
iv. l'incubation dudit second mélange pendant une durée et dans des conditions suffisantes pour que se forment des complexes antigène/anticorps/anticorps ;
v. la détermination de la présence d'anticorps dirigés contre HTLV-I par la détection du signal mesurable ;
b. la détermination de la présence d'anticorps dirigés contre HTI-V-II dans l'échantillon à tester, ladite détermination comprenant :
i. la mise en contact de l'échantillon à tester avec au moins un peptide spécifique de HTLV-II, pour former un mélange ;
ii. l'incubation de ce mélange pendant une durée et dans des conditions suffisantes pour que se forment des complexes antigène/anticorps;
iii. la mise en contact desdits complexes avec un réactif indicateur comprenant un composé générant un signal fixé à un anticorps IgG anti-humain, pour former un second mélange ;
iv. l'incubation dudit second mélange pendant une durée et dans des conditions suffisantes pour que se forment des complexes antigène/anticorps/anticorps ;
v. la détermination de la présence d'anticorps dirigés contre HTLV-II par la détection du signal mesurable ;
c. la détermination du schéma réactionnel de l'échantillon à tester pour les anticorps dirigés contre HTLV-I et contre HTLV-II pour faire la distinction entre les infections à HTLV-I et à HTLV-II,
dans laquelle ledit peptide spécifique de HTLV-II comprend au moins un peptide choisi dans le groupe constitué par le n° ID SEQ 14, le n° ID SEQ 15, le n° ID SEQ 16, le n° ID SEQ 17, le n° ID SEQ 18, le n° ID SEQ 19, le n° ID SEQ 20, le n° ID SEQ 21, le n° ID SEQ 22, le n° ID SEQ 23, le n° ID SEQ 24, le n° ID SEQ 25, le n° ID SEQ 26, le n° ID SEQ 27 et le n° ID SEQ 28.

3. Méthode pour différencier les anticorps dirigés contre HTLV-I des anticorps dirigés contre HTLV-II dans un échantillon à tester, comprenant:
a. la détermination de la présence d'anticorps dirigés contre HTLV-I dans l'échantillon à tester, ladite détermination comprenant :
i. la mise en contact de l'échantillon à tester avec au moins un peptide spécifique de HTLV-I, pour former un mélange ;
ii. l'incubation de ce mélange pendant une durée et dans des conditions suffisantes pour que se forment des complexes antigène/anticorps;
iii. la mise en contact desdits complexes avec un réactif indicateur comprenant un composé générant un signal fixé à un anticorps IgG anti-humain, pour former un second mélange ;
iv. l'incubation dudit second mélange pendant une durée et dans des conditions suffisantes pour que se forment des complexes antigène/anticorps/anticorps ;
v. la détermination de la présence d'anticorps dirigés contre HTLV-I par la détection du signal mesurable ;
b. la détermination de la présence d'anticorps dirigés contre HTLV-II dans l'échantillon à tester, ladite détermination comprenant :
i. la mise en contact de l'échantillon à tester avec au moins un peptide spécifique de HTLV-II, pour former un mélange ;
ii. l'incubation de ce mélange pendant une durée et dans des conditions suffisantes pour que se forment des complexes antigène/anticorps;
iii. la mise en contact desdits complexes avec un réactif indicateur comprenant un composé générant un signal fixé à un anticorps IgG anti-humain, pour former un second mélange ;
iv. l'incubation dudit second mélange pendant une durée et dans des conditions suffisantes pour que se forment des complexes antigène/anticorps/anticorps;
v. la détermination de la présence d'anticorps dirigés contre HTLV-II par la détection du signal mesurable ;
c. la détermination du schéma réactionnel de l'échantillon à tester pour les anticorps dirigés contre HTLV-I et contre HTLV-II pour faire la distinction entre les infections à HTLV-I et à HTLV-II,
dans laquelle ledit peptide spécifique de HTLV-I comprend au moins un peptide choisi dans le groupe constitué par le n° ID SEQ 1, le n° ID SEQ 5 et le n° ID SEQ 7 et dans laquelle ledit peptide spécifique de HTLV-II comprend au moins un peptide choisi dans le groupe constitué par le n° ID SEQ 16, le n° ID SEQ 17, le n° ID SEQ 24 et le n° ID SEQ 27.

4. Peptide spécifique de HTLV-I ayant le n° ID SEQ 1, le n° ID SEQ 5 ou le n° ID SEQ 7.

5. Peptide spécifique de HTLV-II ayant le n° ID SEQ 16, le n° ID SEQ 17, le n° ID SEQ 24 ou le n" ID SEQ 27.

6. Kit d'analyse pour la différenciation de HTLV-I par rapport à HTLV-II, comprenant
un conteneur contenant au moins un peptide spécifique de HTLV-I fixé à une phase solide et
un conteneur contenant au moins un peptide spécifique de HTLV-II fixé à une phase solide,
ledit peptide spécifique de HTLV-I comprenant au moins un peptide choisi dans le groupe constitué par le n° ID SEQ 1, le n° ID SEQ 2, le n° ID SEQ 3, le n° ID SEQ 4, le n° ID SEQ 5, le n° ID SEQ 6, le n° ID SEQ 7, le n° ID SEQ 8, le n° ID SEQ 9, le n° ID SEQ 10, le n° ID SEQ 11, le n° ID SEQ 12 et le n° ID SEQ 13.

7. Kit d'analyse pour la différenciation de HTLV-I par rapport à HTLV-II, comprenant
un conteneur contenant au moins un peptide spécifique de HTLV-I fixé à une phase solide et
un conteneur contenant au moins un peptide spécifique de HTLV-II fixé à une phase solide,
ledit peptide spécifique de HTLV-II comprenant au moins un peptide choisi dans le groupe constitué par le n° ID SEQ 14, le n° ID SEQ 15, le n° ID SEQ 16, le n° ID SEQ 17, le n° ID SEQ 18, le n° ID SEQ 19, le n° ID SEQ 20, le n° ID SEQ 21, le n° ID SEQ 22, le n° ID SEQ 23, le n° ID SEQ 24, le n° ID SEQ 25, le n° ID SEQ 26, le n° ID SEQ 27 et le n° ID SEQ 28.

8. Kit d'analyse pour la différenciation de HTLV-I par rapport à HTLV-II, comprenant
un conteneur contenant au moins un peptide spécifique de HTLV-I fixé à une phase solide et
un conteneur contenant au moins un peptide spécifique de HTLV-II fixé à une phase solide,
ledit peptide spécifique de HTLV-I comprenant au moins un peptide choisi dans le groupe constitué par le n° ID SEQ 1, le n° ID SEQ 5 et le n° ID SEQ 7 et ledit peptide spécifique de HTLV-II comprenant au moins un peptide choisi dans le groupe constitué par le n° ID SEQ 16, le n° ID SEQ 17, le n° ID SEQ 24 et le n° ID SEQ 27.


### SEQUENCE LISTING

### (1) GENERAL INFORMATION:

(i) APPLICANT:
   Shih, Jessie W.
   Burczak, John D.
   Lee, Helen H.
   O'Donnell, Debra L.
(ii) TITLE OF INVENTION: DIFFERENTIATION OF HTLV-I AND HTLV-II USING SYNTHETIC PEPTIDES
(iii) NUMBER OF SEQUENCES: 28
(iv) CORRESPONDENCE ADDRESS:
   (A) ADDRESSEE: Abbott Laboratories
   (B) STREET: One Abbott Park Road
   (C) CITY: Abbott Park
   (D) STATE: Illinois
   (E) COUNTRY: USA
   (F) ZIP: 60064-3500
(v) COMPUTER READABLE FORM:
   (A) MEDIUM TYPE: Floppy disk
   (B) COMPUTER: IBM PC compatible
   (C) OPERATING SYSTEM: PC-DOS/MS-DOS
   (D) SOFTWARE: PatentIn Release #1.0, Version #1.25
(vi) CURRENT APPLICATION DATA:
   (A) APPLICATION NUMBER:
   (B) FILING DATE:
   (C) CLASSIFICATION:
(viii) ATTORNEY/AGENT INFORMATION:
   (A) NAME: Porembski, Priscilla E.
   (B) REGISTRATION NUMBER: 33,207
   (C) REFERENCE/DOCKET NUMBER: 5013.US.01
(ix) TELECOMMUNICATION INFORMATION:
   (A) TELEPHONE: 708-937-4884
   (B) TELEFAX: 708-937-9556

### (2) INFORMATION FOR SEQ ID NO:1:

(i) SEQUENCE CHARACTERISTICS:
   (A) LENGTH: 31 amino acids
   (B) TYPE: amino acid
   (D) TOPOLOGY: linear
(ii) MOLECULE TYPE: peptide
(viii) POSITION IN GENOME:
   (B) MAP POSITION: 174-204
(xi) SEQUENCE DESCRIPTION: SEQ ID NO:1:

Phe Leu Asn Thr Glu Pro Ser Gln Leu Pro Pro Thr Ala Pro Pro Leu
174 175                 180                 185

Leu Pro His Ser Asn Leu Asp His Ile Leu Glu Pro Ser Ile Pro
190                 195                 200

### (2) INFORMATION FOR SEQ ID NO:2:

(i) SEQUENCE CHARACTERISTICS:
   (A) LENGTH: 34 amino acids
   (B) TYPE: amino acid
   (D) TOPOLOGY: linear
(ii) MOLECULE TYPE: peptide
(viii) POSITION IN GENOME:
   (B) MAP POSITION: 180-213
(xi) SEQUENCE DESCRIPTION: SEQ ID NO:2:

Ser Gln Leu Pro Pro Thr Ala Pro Pro Leu Leu Pro His Ser Asn Leu
180                 185                 190                 195

Asp His Ile Leu Glu Pro Ser Ile Pro Trp Lys Ser Lys Leu Leu Thr
                200                 205                 210

Leu Val

### (2) INFORMATION FOR SEQ ID NO:3:

(i) SEQUENCE CHARACTERISTICS:
   (A) LENGTH: 31 amino acids
   (B) TYPE: amino acid
   (D) TOPOLOGY: linear
(ii) MOLECULE TYPE: peptide
(viii) POSITION IN GENOME:
   (B) MAP POSITION: 227-257
(xi) SEQUENCE DESCRIPTION: SEQ ID NO:3:

Cys Ile Asp Arg Ala Ser Leu Ser Thr Trp His Val Leu Tyr Ser Pro
227         230                 235                 240

Asn Val Ser Val Pro Ser Ser Ser Ser Thr Pro Leu Leu Tyr Pro
        245                 250                 255

### (2) INFORMATION FOR SEQ ID NO:4:

(i) SEQUENCE CHARACTERISTICS:
   (A) LENGTH: 31 amino acids
   (B) TYPE: amino acid
   (D) TOPOLOGY: linear
(ii) MOLECULE TYPE: peptide
(viii) POSITION IN GENOME:
   (B) MAP POSITION: 230-260
(xi) SEQUENCE DESCRIPTION: SEQ ID NO:4:

Arg Ala Ser Leu Ser Thr Trp His Val Leu Tyr Ser Pro Asn Val Ser
230                 235                 240                 245

Val Pro Ser Ser Ser Ser Thr Pro Leu Leu Tyr Pro Ser Leu Ala
                250                 255                 260

### (2) INFORMATION FOR SEQ ID NO:5:

(i) SEQUENCE CHARACTERISTICS:
   (A) LENGTH: 24 amino acids
   (B) TYPE: amino acid
   (D) TOPOLOGY: linear
(ii) MOLECULE TYPE: peptide
(viii) POSITION IN GENOME:
   (B) MAP POSITION: 237-260
(xi) SEQUENCE DESCRIPTION: SEQ ID NO:5:

His Val Leu Tyr Ser Pro Asn Val Ser Val Pro Ser Ser Ser Ser Thr
237 240 245 250
Pro Leu Leu Tyr Pro Ser Leu Ala
255 260

### (2) INFORMATION FOR SEQ ID NO:6:

(i) SEQUENCE CHARACTERISTICS:
   (A) LENGTH: 24 amino acids
   (B) TYPE: amino acid
   (D) TOPOLOGY: linear
(ii) MOLECULE TYPE: peptide
(viii) POSITION IN GENOME:
   (B) MAP POSITION: 190-213
(xi) SEQUENCE DESCRIPTION: SEQ ID NO:6:

Leu Pro His Ser Asn Leu Asp His Ile Leu Glu Pro Ser Ile Pro Trp
190                 195                 200                 205

Lys Ser Lys Leu Leu Thr Leu Val
                210

### (2) INFORMATION FOR SEQ ID NO:7:

(i) SEQUENCE CHARACTERISTICS:
   (A) LENGTH: 30 amino acids
   (B) TYPE: amino acid
   (D) TOPOLOGY: linear
(ii) MOLECULE TYPE: peptide
(viii) POSITION IN GENOME:
   (B) MAP POSITION: 100-129
(xi) SEQUENCE DESCRIPTION: SEQ ID NO:7:

Pro Pro Pro Ser Ser Pro Thr His Asp Pro Pro Asp Ser Asp Pro Gln
100                 105                 110                 115

Ile Pro Pro Pro Tyr Val Glu Pro Thr Ala Pro Gln Val Leu
                120                 125

### (2) INFORMATION FOR SEQ ID NO:8:

(i) SEQUENCE CHARACTERISTICS:
   (A) LENGTH: 26 amino acids
   (B) TYPE: amino acid
   (D) TOPOLOGY: linear
(ii) MOLECULE TYPE: peptide
(viii) POSITION IN GENOME:
   (B) MAP POSITION: 104-129
(xi) SEQUENCE DESCRIPTION: SEQ ID NO:8:

Ser Pro Thr His Asp Pro Pro Asp Ser Asp Pro Gln Ile Pro Pro Pro
104 105 110 115

Tyr Val Glu Pro Thr Ala Pro Gln Val Leu
120 125

### (2) INFORMATION FOR SEQ ID NO:9:

(i) SEQUENCE CHARACTERISTICS:
   (A) LENGTH: 21 amino acids
   (B) TYPE: amino acid
   (D) TOPOLOGY: linear
(ii) MOLECULE TYPE: peptide
(viii) POSITION IN GENOME:
   (B) MAP POSITION: 109-129
(xi) SEQUENCE DESCRIPTION: SEQ ID NO:9:

Pro Pro Asp Ser Asp Pro Gln Ile Pro Pro Pro Tyr Val Glu Pro Thr
109 110 115 120

Ala Pro Gln Val Leu
125

### (2) INFORMATION FOR SEQ ID NO:10:

(i) SEQUENCE CHARACTERISTICS:
   (A) LENGTH: 14 amino acids
   (B) TYPE: amino acid
   (D) TOPOLOGY: linear
(ii) MOLECULE TYPE: peptide
(viii) POSITION IN GENOME:
   (B) MAP POSITION: 103-116
(xi) SEQUENCE DESCRIPTION: SEQ ID NO:10:

Ser Ser Pro Thr His Asp Pro Pro Asp Ser Asp Pro Gln Ile
103 105 110 115

### (2) INFORMATION FOR SEQ ID NO:11:

(i) SEQUENCE CHARACTERISTICS:
   (A) LENGTH: 20 amino acids
   (B) TYPE: amino acid
   (D) TOPOLOGY: linear
(ii) MOLECULE TYPE: peptide
(viii) POSITION IN GENOME:
   (B) MAP POSITION: 100-119
(xi) SEQUENCE DESCRIPTION: SEQ ID NO:11:

Pro Pro Pro Ser Ser Pro Thr His Asp Pro Pro Asp Ser Asp Pro Gln
100                 105                 110                 115

Ile Pro Pro Pro

### (2) INFORMATION FOR SEQ ID NO:12:

(i) SEQUENCE CHARACTERISTICS:
   (A) LENGTH: 28 amino acids
   (B) TYPE: amino acid
   (D) TOPOLOGY: linear
(ii) MOLECULE TYPE: peptide
(viii) POSITION IN GENOME:
   (B) MAP POSITION: 100-127
(xi) SEQUENCE DESCRIPTION: SEQ ID NO:12:

Pro Pro Pro Ser Ser Pro Thr His Asp Pro Pro Asp Ser Asp Pro Gln
100                 105                 110                 115

Ile Pro Pro Pro Tyr Val Glu Pro Thr Ala Pro Gln
                120                 125

### (2) INFORMATION FOR SEQ ID NO:13:

(i) SEQUENCE CHARACTERISTICS:
   (A) LENGTH: 27 amino acids
   (B) TYPE: amino acid
   (D) TOPOLOGY: linear
(ii) MOLECULE TYPE: peptide
(viii) POSITION IN GENOME:
   (B) MAP POSITION: 100-126
(xi) SEQUENCE DESCRIPTION: SEQ ID NO:13:

Pro Pro Pro Ser Ser Pro Thr His Asp Pro Pro Asp Ser Asp Pro Gln
100                 105                 110                 115

Ile Pro Pro Pro Tyr Val Glu Pro Thr Ala Pro
                120                 125

### (2) INFORMATION FOR SEQ ID NO:14:

(i) SEQUENCE CHARACTERISTICS:
   (A) LENGTH: 32 amino acids
   (B) TYPE: amino acid
   (D) TOPOLOGY: linear
(ii) MOLECULE TYPE: peptide
(viii) POSITION IN GENOME:
   (B) MAP POSITION: 167-198
(xi) SEQUENCE DESCRIPTION: SEQ ID NO:14:

Pro Leu Trp Phe Ile Thr Ser Glu Pro Thr Gln Pro Pro Pro Thr Ser
167         170                 175                 180
Pro Pro Leu Val His Asp Ser Asp Leu Glu His Val Leu Thr Pro Ser
        185                 190                 195

### (2) INFORMATION FOR SEQ ID NO:15:

(i) SEQUENCE CHARACTERISTICS:
   (A) LENGTH: 32 amino acids
   (B) TYPE: amino acid
   (D) TOPOLOGY: linear
(ii) MOLECULE TYPE: peptide
(viii) POSITION IN GENOME:
   (B) MAP POSITION: 167-198
(xi) SEQUENCE DESCRIPTION: SEQ ID NO:15:

Pro Leu Trp Phe Ile Thr Ser Glu Pro Thr Arg Pro Pro Pro Thr Pro
167 170 175 180
Pro Pro Leu Val His Asp Ser Asp Leu Glu His Val Leu Thr Pro Ser
185 190 195

### (2) INFORMATION FOR SEQ ID NO:16:

(i) SEQUENCE CHARACTERISTICS:
   (A) LENGTH: 28 amino acids
   (B) TYPE: amino acid
   (D) TOPOLOGY: linear
(ii) MOLECULE TYPE: peptide
(viii) POSITION IN GENOME:
   (B) MAP POSITION: 171-198
(xi) SEQUENCE DESCRIPTION: SEQ ID NO:16:

Ile Thr Ser Glu Pro Thr Gln Pro Pro Pro Thr Ser Pro Pro Leu Val
171 175 180 185

His Asp Ser Asp Leu Glu His Val Leu Thr Pro Ser
190 195

### (2) INFORMATION FOR SEQ ID NO:17:

(i) SEQUENCE CHARACTERISTICS:
   (A) LENGTH: 28 amino acids
   (B) TYPE: amino acid
   (D) TOPOLOGY: linear
(ii) MOLECULE TYPE: peptide
(viii) POSITION IN GENOME:
   (B) MAP POSITION: 171-198
(xi) SEQUENCE DESCRIPTION: SEQ ID NO:17:

Ile Thr Ser Glu Pro Thr Arg Pro Pro Pro Thr Pro Pro Pro Leu Val
171 175 180 185

His Asp Ser Asp Leu Glu His Val Leu Thr Pro Ser
190 195

### (2) INFORMATION FOR SEQ ID NO:18:

(i) SEQUENCE CHARACTERISTICS:
   (A) LENGTH: 28 amino acids
   (B) TYPE: amino acid
   (D) TOPOLOGY: linear
(ii) MOLECULE TYPE: peptide
(viii) POSITION IN GENOME:
   (B) MAP POSITION: 173-200
(xi) SEQUENCE DESCRIPTION: SEQ ID NO:18:

Ser Glu Pro Thr Gln Pro Pro Pro Thr Ser Pro Pro Leu Val His Asp Ser
173     175                     180                     185

Asp Leu Glu His Val Leu Thr Pro Ser Thr Ser
190                 195                 200

### (2) INFORMATION FOR SEQ ID NO:19:

(i) SEQUENCE CHARACTERISTICS:
   (A) LENGTH: 32 amino acids
   (B) TYPE: amino acid
   (D) TOPOLOGY: linear
(ii) MOLECULE TYPE: peptide
(viii) POSITION IN GENOME:
   (B) MAP POSITION: 173-204
   (xi) SEQUENCE DESCRIPTION: SEQ ID NO:19:

Ser Glu Pro Thr Gln Pro Pro Pro Thr Ser Pro Pro Leu Val His Asp
173     175                     180                     185

Ser Asp Leu Glu His Val Leu Thr Pro Ser Thr Ser Trp Thr Thr Lys
    190                 195                 200

### (2) INFORMATION FOR SEQ ID NO:20:

(i) SEQUENCE CHARACTERISTICS:
   (A) LENGTH: 34 amino acids
   (B) TYPE: amino acid
   (D) TOPOLOGY: linear
(ii) MOLECULE TYPE: peptide
(viii) POSITION IN GENOME:
   (B) MAP POSITION: 176-209
(xi) SEQUENCE DESCRIPTION: SEQ ID NO:20:

Thr Gln Pro Pro Pro Thr Ser Pro Pro Leu Val His Asp Ser Asp Leu
176             180                 185                 190

Glu His Val Leu Thr Pro Ser Thr Ser Trp Thr Thr Lys Ile Leu Lys
            195                 200                 205

Phe Ile

### (2) INFORMATION FOR SEQ ID NO:21:

(i) SEQUENCE CHARACTERISTICS:
   (A) LENGTH: 34 amino acids
   (B) TYPE: amino acid
   (D) TOPOLOGY: linear
(ii) MOLECULE TYPE: peptide
(viii) POSITION IN GENOME:
   (B) MAP POSITION: 176-209
(xi) SEQUENCE DESCRIPTION: SEQ ID NO:21:

Thr Arg Pro Pro Pro Thr Pro Pro Pro Leu Val His Asp Ser Asp Leu
176 180 185 190

Glu His Val Leu Thr Pro Ser Thr Ser Trp Thr Thr Lys Met Leu Lys
195 200 205

Phe Ile

### (2) INFORMATION FOR SEQ ID NO:22:

(i) SEQUENCE CHARACTERISTICS:
   (A) LENGTH: 30 amino acids
   (B) TYPE: amino acid
   (D) TOPOLOGY: linear
(ii) MOLECULE TYPE: peptide
(viii) POSITION IN GENOME:
   (B) MAP POSITION: 228-257
(xi) SEQUENCE DESCRIPTION: SEQ ID NO:22:

Ser Leu Ser Ser Trp His Val Leu Tyr Thr Pro Asn Ile Ser Ile Pro
228     230                     235                     240

Gln Gln Thr Ser Ser Arg Thr Ile Leu Phe Pro Ser Leu Ala
    245                 250                 255

### (2) INFORMATION FOR SEQ ID NO:23:

(i) SEQUENCE CHARACTERISTICS:
   (A) LENGTH: 10 amino acids
   (B) TYPE: amino acid
   (D) TOPOLOGY: linear
(ii) MOLECULE TYPE: peptide
(viii) POSITION IN GENOME:
   (B) MAP POSITION: 186-195
(xi) SEQUENCE DESCRIPTION: SEQ ID NO:23:

Val His Asp Ser Asp Leu Glu His Val Leu
186 190 195

### (2) INFORMATION FOR SEQ ID NO:24:

(i) SEQUENCE CHARACTERISTICS:
   (A) LENGTH: 26 amino acids
   (B) TYPE: amino acid
   (D) TOPOLOGY: linear
(ii) MOLECULE TYPE: peptide
(viii) POSITION IN GENOME:
   (B) MAP POSITION: 83-108
(xi) SEQUENCE DESCRIPTION: SEQ ID NO:24:

Trp Ile Lys Lys Pro Asn Arg Gln Gly Leu Gly Tyr Tyr Ser Pro Ser
83      85                      90                      95
Tyr Asn Asp Pro Cys Ser Leu Gln Cys Pro
    100                 105

### (2) INFORMATION FOR SEQ ID NO:25:

(i) SEQUENCE CHARACTERISTICS:
   (A) LENGTH: 21 amino acids
   (B) TYPE: amino acid
   (D) TOPOLOGY: linear
(ii) MOLECULE TYPE: peptide
(viii) POSITION IN GENOME:
   (B) MAP POSITION: 115-135
(xi) SEQUENCE DESCRIPTION: SEQ ID NO:25:

Pro Pro Ser Pro Glu Ala His Val Pro Pro Pro Tyr Val Glu Pro Thr
115                 120                 125                 130

Thr Thr Gln Cys Phe
                135

### (2) INFORMATION FOR SEQ ID NO:26:

(i) SEQUENCE CHARACTERISTICS:
   (A) LENGTH: 19 amino acids
   (B) TYPE: amino acid
   (D) TOPOLOGY: linear
(ii) MOLECULE TYPE: peptide
(viii) POSITION IN GENOME:
   (B) MAP POSITION: 111-129
(xi) SEQUENCE DESCRIPTION: SEQ ID NO:26:

Pro Pro Pro Pro Pro Pro Ser Pro Glu Ala His Val Pro Pro Pro Tyr
111 115 120 125

Val Glu Pro

### (2) INFORMATION FOR SEQ ID NO:27:

(i) SEQUENCE CHARACTERISTICS:
   (A) LENGTH: 19 amino acids
   (B) TYPE: amino acid
   (D) TOPOLOGY: linear
(ii) MOLECULE TYPE: peptide
(viii) POSITION IN GENOME:
   (B) MAP POSITION: 109-127
(xi) SEQUENCE DESCRIPTION: SEQ ID NO:27:

Thr Pro Pro Pro Pro Pro Pro Pro Ser Pro Glu Ala His Val Pro Pro
109 110 115 120

Pro Tyr Val
125

### (2) INFORMATION FOR SEQ ID NO:28:

(i) SEQUENCE CHARACTERISTICS:
   (A) LENGTH: 19 amino acids
   (B) TYPE: amino acid
   (D) TOPOLOGY: linear
(ii) MOLECULE TYPE: peptide
(viii) POSITION IN GENOME:
   (B) MAP POSITION: 107-125
(xi) SEQUENCE DESCRIPTION: SEQ ID NO:28:

Thr Thr Thr Pro Pro Pro Pro Pro Pro Pro Ser Pro Glu Ala His Val
107 110 115 120
Pro Pro Pro
125